# EUROPEAN PATENT APPLICATION

(11) **EP 4 206 316 A1**
(43) Date of publication of application: **05.07.2023**
(21) Application number: 21861933.6
(22) Date of filing: 09.08.2021
(51) Int. Cl.: C12N 1/20, A61K 35/747, A61P 35/00, A23L 33/135, C12R 1/25

(54) **LACTOBACILLUS PLANTARUM MICROORGANISMS HAVING ANTI-CANCER ACTIVITY, COMPOSITION INCLUDING SAME, AND METHOD FOR PREVENTING OR TREATING CANCER USING SAME**

(30) Priority: 31.08.2020 KR 20200110598
(71) Applicant: LIVEOME Inc., Yeongtong-gu, Suwon-si Gyeonggi-do 16506 (KR)
(72) Inventor: KIM, Yeung Hyen, Hwaseong-si, Gyeonggi-do 18423 (KR); KWON, Do Hyeong, Suwon-si, Gyeonggi-do 16509 (KR); PARK, Kwang Seo, Suwon-si, Gyeonggi-do 16509 (KR); DONG, Hye Jin, Incheon 21994 (KR); JANG, Hye Jeong, Seoul 06626 (KR); SONG, Ji Yoon, Seongnam-si, Gyeonggi-do 13479 (KR)
(74) Representative: Brevalex
(86) International application number: PCT/KR2021/010524
(87) International publication number: WO 2022/045636

(57) **Abstract**

Provided are a microorganism as *Lactobacillus plantarum* having anti-cancer activity, a composition including the same, and a method of preventing or treating a cancer by using the same.

## Description

### Technical Field

The present invention relates to a *Lactobacillus plantarum* microorganism having anti-cancer activity, a composition including the same, and prevention or treatment of cancer by using the same.

### Background Art

*Lactobacillus plantarum* belongs to the genus *Lactobacillus* and is frequently found in fermented food products and anaerobic plant materials. *L. plantarum* is a grampositive, rod-shaped bacterium. *L. plantarum* is a straight rod with a rounded tip and typically with a width of 0.9 um to 1.2 um and a length of 3 um to 8 um. *L. plantarum* can grow at a pH of 3.4 to 8.8 and at a temperature of 12 °C to 40 °C.

*L. plantarum* helps maintain antioxidant activity and intestinal permeability. Also, *L. plantarum* is effective in treating irritable bowel syndrome (IBS). For example, KR10-2011-0000871 discloses *Lactobacillus plantarum* HY7711 with antioxidant activity.

Meanwhile, in a cancer treatment, an immuno-anticancer drug has a mechanism of attacking cancer cells through immune activation, but only 20 % to 30% of target patients respond to the drug, and thus there is still a need for efficient immuno-anticancer drugs.

### Disclosure

### Technical Problem

One aspect provides a microorganism as *Lactobacillus plantarum* LMT17-31 (Accession No.: KCTC-14283BP) having anti-tumor activity, or a culture or extract thereof.

Another aspect provides a pharmaceutical composition for preventing or treating a cancer comprising, as an active ingredient, the microorganism or a culture or extract thereof.

Another aspect provides a food composition for preventing or ameliorating a cancer comprising, as an active ingredient, the microorganism or a culture or extract thereof.

Another aspect provides a method of preventing or treating a cancer in a subject, the method comprising administering to the subject an effective amount of the microorganism or a culture or extract thereof for the cancer treatment.

### Technical Solution

A first aspect provides a microorganism as *Lactobacillus plantarum* LMT17-31 (Accession No.: KCTC-14283BP) having anti-tumor activity, or a culture or extract thereof.

The microorganism or a culture or extract thereof may inhibit tumor growth. The terms "cancer" and "tumor" are used interchangeably herein. The microorganism or a culture or extract may increase a level of immune cells in a tumor. The microorganism or a culture or extract thereof may increase a level of CD8 T cells in a tumor. The microorganism or a culture or extract may increase infiltration of immune cells into a tumor. The immune cells may be CD8 T cells, CD4 T cells, NK cells, B cells, dendritic cells, macrophages, neutrophils, and the like. The microorganism or a culture or extract may activate the immune cells. The activation may include promotion of production, secretion, or both production and secretion of cytokines or enzymes having anti-tumor activity. The cytokines or enzymes may include at least one of interferon gamma and granzyme B. The microorganism may be excellent in acid resistance, bile resistance, and intestinal adherence. The microorganism may be isolated from rice wine.

The acid resistance may refer to a survival rate of 40 % or more, 45 % or more, 50 % or more, or 60 % or more, or a survival rate in a range of 80 % to 90 %, 80 % to 95 %, 85 % to 90 %, or 90 % to 95%, when the microorganism is cultured in an MRS medium under conditions of a pH of 2.5 and a temperature of 37 °C for 2 hours.

The bile acid resistance may refer to a survival rate of 65 % or more, 70 % or more, 75 % or more, 80 % or more, 90 % or more, or 95 % or more, or a survival rate in a range of 75 % to 90 %, 75 % to 95 %, 80 % to 90 %, 80 % to 95 %, 85 % to 90 %, or 90 % to 95 %, when the microorganism is cultured in a 0.3 % bile acid-containing MRS under conditions of a temperature of 37 °C for 2 hours.

The microorganism or a culture or extract may promote infiltration of CD8 T cells into a tumor. The promotion may refer to an increase in the percentage of the number of CD8 T cells to the number of T cells in a tumor, compared to a case where the microorganism or a culture or extract is not present, by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, or 100 % or more, or by a range of 5 % to 100 %, 10 % to 100 %, 20 % to 100 %, 30 % to 100 %, 40 % to 100 %, 50 % to 100 %, 60 % to 100 %, 70 % to 100 %, 80 % to 100 %, or 90 % to 100 %.

The microorganism or a culture or extract thereof may promote production, secretion, or both production and secretion of at least one of interferon gamma and granzyme B of tumor-infiltrating CD8 T cells. The promotion may refer to an increase in the percentage of the number of interferon gamma-producing cells to CD8 T cells, compared to a case where the microorganism or a culture or extract is not present, by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, or 100 % or more, or by a range of 5 % to 100 %, 10 % to 100 %, 20 % to 100 %, 30 % to 100 %, 40 % to 100 %, 50 % to 100 %, 60 % to 100 %, 70 % to 100 %, 80 % to 100 %, or 90 % to 100 %. In addition, the promotion may refer to an increase in the percentage of the number of granzyme B-producing cells to CD8 T cells, compared to a case where the microorganism or a culture or extract is not present, by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, or 100 % or more, or by a range of 5 % to 100%, 10 % to 100 %, 20 % to 100 %, 30% to 100 %, 40 % to 100 %, 50 % to 100 %, 60 % to 100 %, 70 % to 100 %, 80 % to 100 %, or 90 % to 100 %.

The microorganism or a culture or extract may inhibit the tumor growth by CD8 T cells. The inhibition may refer to a decrease in the size of a tumor, compared to a case where the microorganism or a culture or extract is not present, by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, or 100 % or more, or by a range of 5 % to 100 %, 10 % to 100 %, 20 % to 100 %, 30 % to 100 %, 40 % to 100 %, 50 % to 100 %, 60 % to 100 %, 70 % to 100 %, 80 % to 100 %, or 90 % to 100 %.

A second aspect provides a pharmaceutical composition for preventing or treating a cancer comprising, as an active ingredient, the microorganism or a culture or extract thereof.

In the pharmaceutical composition, the cancer may be a solid cancer. The cancer may be a solid cancer that is not located in the intestines. The cancer may be a metastatic cancer. Non-limiting examples of the cancer include breast cancer, lung cancer, head or neck cancer, colorectal cancer, esophageal cancer, laryngeal cancer, stomach cancer, liver cancer, pancreatic cancer, bone cancer, skin cancer, skin or intraocular melanoma, uterine cancer, ovarian cancer, rectal cancer, perianal cancer, colon cancer, breast cancer, fallopian tube carcinoma, endometrial carcinoma, cervical carcinoma, vaginal carcinoma, vulvar carcinoma, Hodgkin's disease, small intestine cancer, endocrine cancer, thyroid cancer, parathyroid cancer, adrenal cancer, soft tissue sarcoma, urethral cancer, penile cancer, prostate cancer, lymphocytic lymphoma, bladder cancer, renal or ureteric cancer, renal cell carcinoma, renal pelvic carcinoma, CNS tumor, primary CNS lymphoma, spinal cord tumor, brainstem glioma, and pituitary adenoma, but are not limited thereto.

In the pharmaceutical composition, the pharmaceutical composition may inhibit the cancer growth.

In the pharmaceutical composition, the pharmaceutical composition may be administered in combination with an immune checkpoint inhibitor. The immune checkpoint inhibitor may be administered before, concurrently with, or after administration of the microorganism or a culture or extract thereof.

In an embodiment, the immune checkpoint inhibitor may be at least one selected from the group consisting of an anti-PD-1 antibody, an anti-PD-L1 antibody, an anti-PD-L2 antibody, an anti-CTLA-4 antibody, an anti-TIM-3 antibody, an anti-LAG3 antibody, an anti-IDO1 antibody, an anti-TIGIT antibody, an anti-B7H3 antibody, an anti-B7H4 antibody, an anti-BTLA antibody, and an anti-B7H6 antibody, and an antigen-linkage fragment thereof. In one or more embodiments, the immune checkpoint inhibitor may be at least one selected from the group consisting of ipilimumab (Yervoy^{®}, BMS/Ono), tremelimumab (AstraZeneca), atezolizumab (Tecentriq^{®}, Roche), nivolumab (Opdivo^{®}, BMS/Ono), pembrolizumab (Keytruda^{®}, MSD), avelumab (Bavencio^{®}, Pfizer/Merck, Germany), durvalumab (Imfinzi^{®}, AstraZeneca/Medlmmune), and an antigen-binding fragment thereof, but is not limited thereto.

In the pharmaceutical composition, the pharmaceutical composition may be administered in combination with a chemotherapeutic agent. The chemotherapeutic agent may be administered before, concurrently with, or after administration of the microorganism or a culture or extract thereof.

Non-limiting examples of the chemotherapeutic agent are an alkylating agent, a nitrosourase, an antimetabolite, an anticancer antibiotic, a plant-derived alkaloid, a topoisomerase inhibitor, a hormone drug, a hormone antagonist, a leukopenia, such as a therapeutic agent for neutropenia, a therapeutic agent for thrombocytopenia, an antiemetic drug, an aromatase inhibitor, a P-glycoprotein inhibitor, a platinum complex derivative, other immunotherapeutic drugs, and other anti-cancer drugs. Examples of a cytotoxic agent that can be co-administered may include an anti-microtubule agent, a topoisomerase inhibitor, an antimetabolite, a mitotic inhibitor, an alkylating agent, an anthracycline, a vinca alkaloid, an intercalating agent, an agonist that interferes with a signal transduction pathway, an agonist that promotes apoptosis, a proteosome inhibitor, and radiation such as local or systemic irradiation of radiation. Non-limiting examples of an additional therapeutic agent include a peptide, a polypeptide, a protein, a fusion protein, a nucleic acid molecule, a small molecule, a mimetic agonist, a synthetic drug, an inorganic molecule, and an organic molecule, but are not limited thereto.

In the pharmaceutical composition, the pharmaceutical composition may include a pharmaceutically acceptable carrier. The carrier may be a stabilizer, an excipient, a diluent, or an auxiliary agent. The carrier may be, for example, any and all aqueous and non-aqueous solutions, sterile solutions, solvents, buffers, such as phosphate buffered saline (PBS) solutions, water, suspensions, emulsions, such as oil/water emulsions, various types of wetting agents, liposomes, dispersion media, and coatings that are suitable for pharmaceutical administration, particularly oral administration. Use of such media and agonists in the pharmaceutical composition is well known in the art, and the pharmaceutical composition including the carrier may be formulated by methods well known in the art.

The pharmaceutical composition may include the microorganism or a culture or extract thereof in a "therapeutically effective amount". In the pharmaceutical composition, the "therapeutically effective amount" refers to an amount sufficient to exhibit a therapeutic effect when administered to a subject in need of treatment by administration once or at least two times. The term "treatment" refers to treating a cancer disease or a medical symptom of a cancer in a subject, such as a mammal including a human, and includes the following: alleviation of a cancer disease or a medical symptom of cancer; inhibition of a cancer disease or a medical symptom of cancer, i.e., slowing or stopping of progression of a disease or a medical symptom of cancer in a subject; or reduction of a cancer disease or a medical symptom of cancer in a subject. The term "effective amount" may be appropriately selected by a person skilled in the art. The term "effective amount" may be in a range of 0.01 wt% to 50 wt% or 0.1 wt% to 20 wt%, based on the weight of the pharmaceutical composition. In addition, the pharmaceutical composition may be administered at a concentration of 1×10⁶ CFU/g or more, 1×10⁷ CFU/g or more, 1×10⁸ CFU/g or more, or 1×10⁹ CFU/g or more, based on the weight of the pharmaceutical composition. For example, a bacterial strain may be administered at a concentration of 1×10¹⁵ CFU/g or less, 1×10¹⁴ CFU/g or less, 1×10¹³ CFU/g or less, or 1×10¹² CFU/g or less. For example, a bacterial strain may be administered at a concentration in a range of 1×10⁶ CFU/g to 1×10¹⁵ CFU/g, 1×10⁷ CFU/g to 1×10¹⁴ CFU/g, 1×10⁸ CFU/g to 1×10¹³ CFU/g, 1×10⁹ CFU/g to 1×10¹² CFU/g, or 1×10¹⁰ CFU/g to 1×10¹² CFU/g.

The pharmaceutical composition may be administered orally. In this regard, the pharmaceutical composition may be formulated in various forms, such as a tablet, a capsule, an aqueous solution, a dry syrup, a suspension, and the like. In the case of a tablet for oral use, an excipient, such as lactose, corn starch, and like, and a lubricant, such as magnesium stearate, may be typically added. In the case of a capsule for oral administration, lactose and/or dried corn starch may be used as a diluent. When an aqueous suspension for oral use is required, an active ingredient may be combined with an emulsifier and/or a suspending agent. When needed, a specific sweetening and/or a flavoring agent may be added. In an embodiment, the pharmaceutical composition may be a formulation that allows the microorganism or a culture or extract thereof to be stabilized in an acidic environment such as gastric juice. For example, the pharmaceutical composition may be a capsule containing the microorganism or a culture or extract thereof, or a tablet coated with the microorganism or a culture or extract thereof as a film.

A third aspect provides a food composition for preventing or ameliorating a cancer comprising, as an active ingredient, the microorganism or a culture or extract thereof.

The food composition may include a sitologically acceptable carrier. The carrier may be a stabilizer, an excipient, a diluent, or an auxiliary agent. The carrier may be, for example, any and all aqueous and non-aqueous solutions, sterile solutions, solvents, buffers, such PBS solutions, water, suspensions, emulsions, such as oil/water emulsions, various types of wetting agents, liposomes, dispersion media, and coatings that are suitable for pharmaceutical administration, particularly oral administration. Use of such media and agonists in the pharmaceutical composition is well known in the art, and the pharmaceutical composition including the carrier may be formulated by methods well known in the art.

The food may be a dairy product, a soy product, a vegetable and fruit product, or a food additive. The dairy product may be fermented milk, butter, cheese, or powdered milk. The food may be a health functional food. The health functional food may be a health functional food for preventing or improving a cancer. The food may also be a beverage, a confectionery, a diet bar, a chocolate, a pizza, a ramen, other noodles, a chewing gum, and an ice cream.

The food may include an ingredient commonly added during food preparation, and for example, include a protein, a carbohydrate, a fat, a nutrient, a seasoning agent, and a flavoring agent.

Examples of the carbohydrate used for food preparation include: a monosaccharide, such as glucose, fructose, and the like; a disaccharide, such as maltose, sucrose, oligosaccharide, and the like; and a polysaccharide, such as conventional sugar including dextrin and cyclodextrin, and sugar alcohol including xylitol, sorbitol, erythritol, and the like. Also, the flavoring agent may be a natural flavoring agent and a synthetic flavoring agent such as saccharin and aspartame. The natural flavoring agent may be a stevia extract such as thaumatin, rebaudioside A, and glycyrrhizin. The health functional food refers to food bringing a specific effect on health when ingested.

In the food composition, the amount of the microorganism may be in a range of 0.01 wt% to 50 wt% or 0.1 wt% to 20 wt%, based on the weight of the food composition. In addition, the food composition may be administered at a concentration of 1×10⁶ CFU/g or more, 1×10⁷ CFU/g or more, 1×10⁸ CFU/g or more, or 1×10⁹ CFU/g or more, based on the weight of the food composition. For example, a bacterial strain may be administered at a concentration 1×10¹⁵ CFU/g or less, 1×10¹⁴ CFU/g or less, 1×10¹³ CFU/g or less, or 1×10¹² CFU/g or less. For example, a bacterial strain may be administered at a concentration in a range of 1×10⁶ CFU/g to 1×10¹⁵ CFU/g, 1×10⁷ CFU/g to 1×10¹⁴ CFU/g, 1×10⁸ CFU/g to 1×10¹³ CFU/g, 1×10⁹ CFU/g to 1×10¹² CFU/g, or 1×10¹⁰ CFU/g to 1×10¹² CFU/g.

A fourth aspect provides a method of preventing or treating a cancer in a subject, the method comprising administering to the subject an effective amount of the microorganism or a culture or extract thereof for the cancer treatment

The subject may be a mammal. The mammal may be a human or a non-human mammal. The administration may be oral administration.

The term "amount effective to treat a cancer" refers to an amount effective to prevent or treat a cancer. The "amount effective to treat a cancer" may be in a range of 0.01 mg to 200 mg of the microorganism or a culture or extract thereof per kilogram (kg) of body weight of the subject, or 0.1 mg to 400 mg of the microorganism or a culture or extract thereof per kg of body weight of the subject. In addition, the "amount effective to treat a cancer" may be administered at a concentration of 1×10⁶ CFU or more, 1×10⁷ CFU or more, 1×10⁸ CFU or more, or 1×10⁹ CFU or more per subject. For example, a bacterial strain may be administered at a concentration of 1×10¹⁵ CFU or less, 1×10¹⁴ CFU or less, 1×10¹³ CFU or less, or 1×10¹² CFU or less. For example, a bacterial strain may be administered at a concentration in a range of 1×10⁶ CFU to 1×10¹⁵ CFU, 1×10⁷ CFU to 1×10¹⁴ CFU, 1×10⁸ CFU to 1×10¹³ CFU, 1×10⁹ CFU to 1×10¹² CFU, or 1×10¹⁰ CFU to 1×10¹² CFU. For example, a bacterial strain as an active ingredient may be administered in divided doses twice a day or several times a day.

The method may include administering the microorganism or a culture or extract thereof in combination with an immune checkpoint inhibitor. The immune checkpoint inhibitor may be administered before, concurrently with, or after administration of the microorganism or a culture or extract thereof.

In an embodiment, the immune checkpoint inhibitor may be at least one selected from the group consisting of an anti-PD-1 antibody, an anti-PD-L1 antibody, an anti-PD-L2 antibody, an anti-CTLA-4 antibody, an anti-TIM-3 antibody, an anti-LAG3 antibody, an anti-IDO1 antibody, an anti-TIGIT antibody, an anti-B7H3 antibody, an anti-B7H4 antibody, an anti-BTLA antibody, and an anti-B7H6 antibody, and an antigen-linkage fragment thereof. In one or more embodiments, the immune checkpoint inhibitor may be at least one selected from the group consisting of ipilimumab (Yervoy^{®}, BMS/Ono), tremelimumab (AstraZeneca), atezolizumab (Tecentriq^{®}, Roche), nivolumab (Opdivo^{®}, BMS/Ono), pembrolizumab (Keytruda^{®}, MSD), avelumab (Bavencio^{®}, Pfizer/Merck, Germany), durvalumab (Imfinzi^{®}, AstraZeneca/Medlmmune), and an antigen-binding fragment thereof, but is not limited thereto.

The method may include administering the microorganism or a culture or extract thereof in combination with a chemotherapeutic agent. The chemotherapeutic agent may be administered before, concurrently with, or after administration of the microorganism or a culture or extract thereof.

Non-limiting examples of the chemotherapeutic agent include an alkylating agent, a nitrosourase, an antimetabolite, an anticancer antibiotic, a plant-derived alkaloid, a topoisomerase inhibitor, a hormone drug, a hormone antagonist, a leukopenia, such as a therapeutic agent for neutropenia, a therapeutic agent for thrombocytopenia, an antiemetic drug, an aromatase inhibitor, a P-glycoprotein inhibitor, a platinum complex derivative, other immunotherapeutic drugs, and other anti-cancer drugs. Examples of a cytotoxic agent that can be co-administered may include an anti-microtubule agent, a topoisomerase inhibitor, an antimetabolite, a mitotic inhibitor, an alkylating agent, an anthracycline, a vinca alkaloid, an intercalating agent, an agonist that interferes with a signal transduction pathway, an agonist that promotes apoptosis, a proteosome inhibitor, and radiation such as local or systemic irradiation of radiation. Non-limiting examples of an additional therapeutic agent include a peptide, a polypeptide, a protein, a fusion protein, a nucleic acid molecule, a small molecule, a mimetic agonist, a synthetic drug, an inorganic molecule, and an organic molecule, but are not limited thereto.

### Advantageous Effects

A microorganism as *Lactobacillus plantarum* LMT17-31 (Accession No.: KCTC-14283BP) having anti-tumor activity, or a culture or extract thereof according to an aspect may be used to prevent or treat a cancer.

A pharmaceutical composition for preventing or treating a cancer according to another aspect may be used to prevent or treat a cancer.

A food composition for preventing or ameliorating a cancer according to another aspect may be used to prevent or ameliorate a cancer.

A method of preventing or treating a cancer in a subject according to another aspect may be used to prevent or treat a cancer efficiently.

### Description of Drawings

FIG. 1 shows representative optical microphotographs of a selected *Lactobacillus plantarum* LMT17-31 strain and a KCTC3108 strain as a type strain.
FIGS. 2A and 2B are diagrams showing results of measuring size of a tumor after the *L. plantarum* LMT17-31 strain is administered to mice having a tumor.
FIGS. 3A, 3B, 3C, 3D, 3E, and 3F are diagrams showing results of analyzing CD8 T cells infiltrating into a tumor and secretion of cytokines therefrom after the *L. plantarum* LMT17-31 strain is administered to mice having a tumor.

### Best Mode

Hereinafter, the present invention will be described in detail with reference to Examples below. However, these Examples are for illustrative purposes only, and the scope of the present invention is not intended to be limited by these Examples.

### Example 1: Isolation and identification of Lactobacillus plantarum LMT17-31 strain

### 1. Isolation of L. plantarum strain

An *L. plantarum strain* was isolated from a rice wine sample. First, the sample was spread on an MRS medium (Difco, USA) and cultured anaerobically at 30 °C. As a pretreatment method of the sample, the sample was obtained aseptically and diluted with 180 ml of a 0.85 % NaCl solution, and then, a stock solution of the rice wine was homogenized with a stomacher for 5 minutes. The homogenized sample was diluted by stages in a tube containing 9 ml of a sterilized 0.85 % NaCl solution to prepare a rice wine sample. The rice wine sample was spread on an MRS plate medium (Difco, USA) and cultured at 37 °C for 2 days to 3 days. Then, colonies that appeared thereon were sorted according to shape and color, and were purely isolated again.

### 2. Identification of L. plantarum strain

### (1) Analysis of morphological characteristics

The selected *L. plantarum* strain was cultured in an MRS plate medium (Difco, USA), and morphological characteristics of the colonies thereof were observed. The morphological characteristics of the colonies appeared in the MRS plate medium for the selected *L. plantarum* strain and the KCTC3108 type strain of the *L. plantarum* are shown in Table 1 below.

**Table 1**

| | LMT17-31 | KCTC3108 |
|---|---|---|
| Shape | Circular | Circular |
| Size | 1 mm | 1 mm |
| Color | Cream | Cream |
| Transparency | Opaque | Opaque |
| Protuberance | Convex | Convex |
| Surface | Smooth | Smooth |
| Aerobic growth and development | + | + |
| Anaerobic growth and development | + | + |

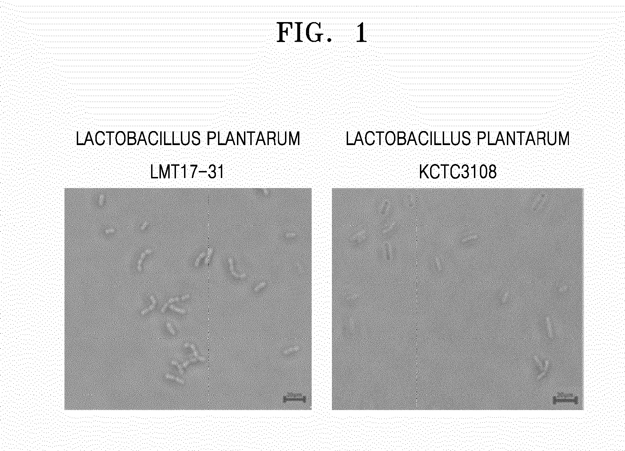

FIG. 1 shows representative optical microphotographs of the selected *L*. *plantarum* LMT17-31 strain and the KCTC3108 strain. As shown in FIG. 1, the LMT17-31 strain was a rod-shaped bacillus and was similar in appearance to the typical genus *Lactobacillus.*

### (2) 16S rDNA analysis

The 16S rRNA gene of the isolated LMT17-31 strain was amplified, and the nucleotide sequence of the amplified 16S rRNA gene was analyzed. The amplification was performed by PCR using the genomic DNA of the LMT17-31 strain as a template and an oligonucleotide of SEQ ID NO: 1 and an oligonucleotide of SEQ ID NO: 2 (Macrogen Inc.) as primer sets. The nucleotide sequence of the 16S rDNA of the isolated LMT17-31 strain is shown as a sequence of SEQ ID NO: 3. The nucleotide sequence of the identified 16S rDNA was compared with the nucleotide sequence of the known 16S rDNA by using NCBI blast (http://www.ncbi.nlm.nih.gov/). As a result, the 16S rDNA of LMT17-31 was found to have 100 % sequence identity to the *L. plantarum* species. In addition, as a result of phylogenetic tree analysis, LMT17-31 was found to be the same as the *L. plantarum* species. As a result, the LMT17-31 strain was identified as a new strain belonging to a newly found *L. plantarum* species.

The present inventors named LMT17-31 lactic acid bacteria as *"L. plantarum* LMT17-31" (Accession number: KCTC 14283BP), which was deposited in the Korean Collection for Type Cultures (KCTC) of the Korea Research Institute of Bioscience and Biotechnology on August 26, 2020.

### Example 2: Analysis of physiological activity characteristics of L. plantarum LMT17-31 strain

### 1. Sugar fermentation characteristics of L. plantarum LMT17-31 strain

The sugar metabolism characteristics of the selected LMT17-31 strain were verified with the API 50 CHL kit (BioMetrieux, France) according to the experimental method provided by the supplying company. Table 2 shows the sugar fermentation characteristics of the identified LMT17-31 strain.

**Table 2**

| No. | Carbohydrate | LMT17-31 | |
|---|---|---|---|
| | | 24 hours | 48 hours |
| 1 | Glycerol | - | - |
| 2 | Erythritol | - | - |
| 3 | D-arabinose | - | - |
| 4 | L-arabinose | + | + |
| 5 | D-ribose | + | + |
| 6 | D-xylose | - | - |
| 7 | L-xylose | - | - |
| 8 | D-adonitol | - | - |
| 9 | Methyl-βD-xylopyranoside | - | - |
| 10 | D-galactose | + | + |
| 11 | D-glucose | + | + |
| 12 | D-fructose | + | + |
| 13 | D-mannose | + | + |
| 14 | L-sorbose | - | - |
| 15 | L-rhamnose | - | - |
| 16 | Dulcitol | - | - |
| 17 | Inositol | - | - |
| 18 | Mannitol | + | + |
| 19 | D-sorbitol | + | + |
| 20 | Methyl αD-mannopyranoside | + | + |
| 21 | Methyl αD-glucopyranoside | + | + |
| 22 | N-acetylglucosamine | + | + |
| 23 | Amygdalin | + | + |
| 24 | Arbutin | + | + |
| 25 | Esculin | + | + |
| 26 | Salicin | + | + |
| 27 | D-cellobiose | + | + |
| 28 | D-maltose | + | + |
| 29 | D-lactose | + | + |
| 30 | D-melibiose | + | + |
| 31 | D-sucrose | + | + |
| 32 | D-trehalose | + | + |
| 33 | Inulin | - | - |
| 34 | D-melezitose | + | + |
| 35 | D-raffinose | + | + |
| 36 | Amidon | - | - |
| 37 | Glycogen | - | - |
| 38 | Xylitol | - | - |
| 39 | Gentibiose | + | + |
| 40 | D-turanose | + | + |
| 41 | D-lyxose | - | - |
| 42 | D-tagatose | - | - |
| 43 | D-fucose | - | - |
| 44 | L-fucose | - | - |
| 45 | D-arabitol | - | - |
| 46 | L-arabitol | - | - |
| 47 | Potassium gluconate | + | + |
| 48 | Potassium 2-cetogluconate | - | - |
| 49 | Potassium 5-ketogluconate | - | - |

### 2. Evaluation of stability of L. plantarum LMT17-31 strain

### (1) Investigation of acid resistance

To evaluate the acid resistance of the *L. plantarum* LMT17-31 strain, an experiment was conducted as follows. The LMT17-31 strain was inoculated into a sterilized MRS broth and cultured at 37 °C for 16 hours. Then, 1 % of the strain was inoculated into a different sterilized MRS broth having a pH of 2.5 adjusted by HCl, and cultured at 37 °C for 2 hours. The samples were collected immediately after the strain inoculation and after 2 hours of the incubation, diluted in a fresh MRS broth, spread on an MRS plate medium, and cultured at 37 °C for 24 hours. Then, the number of colonies on the MRS plate medium was counted to measure the number of bacteria.

As shown in Table 3, the *L. plantarum* LMT17-31 strain showed 44.2 % acid resistance to the acidity of pH 2.5, and the KCTC3108 type strain had 67.1 % acid resistance to the same acidity.

**Table 3**

| | **LMT17-31** | **KCTC3108** |
|---|---|---|
| MRS (pH 6.8) (number of cells/plate) | 2.2x10⁹ | 7.6x10⁸ |
| MRS (pH 2.5) (number of cells/plate) | 1.0x10⁹ | 5.1x10⁸ |
| Survival rate (%) | 44.2 | 67.1 |

### (2) Investication of bile resistance

To confirm the influence of bile acid on the growth of the *L. plantarum* LMT17-31 strain, an experiment was conducted as follows. The selected strain was inoculated into a sterilized MRS broth and cultured at 37 °C for 24 hours. Considering that the concentration of bile salts in the intestinal tract is about 0.1 %, an MRS broth containing 0.3% of bile salts (Sigma, USA) was prepared and 1 % of the strain was inoculated thereinto to be cultured at 37 °C for 2 hours. The samples were collected immediately after the strain inoculation and after 2 hours of the incubation, diluted in a fresh MRS broth, spread on an MRS plate medium, and cultured at 37 °C for 24 hours. Then, the number of colonies on the MRS plate medium was counted to measure the number viable strain cells. As a control group, the incubation was performed in the same manner in an MRS broth without 0.3 % bile acid, and the number of viable strain cells was measured. Table 4 shows the results of measuring the bile salt resistance.

As shown in Table 4, since the LMT17-31 strain maintained a survival rate of 66.4 % at a concentration of 0.3 %, which is a concentration higher than 0.1 % that is similar to the actual concentration in the intestine, the LMT17-31 strain can be a basis sufficiently enough for the survival in the intestines of humans or animals.

**Table 4**

| | LMT17-31 | KCTC3108 |
|---|---|---|
| Control group (number of cells/plate) | 2.2x10⁹ | 7.6x10⁸ |
| 0.3 % bile salts (number of cells/plate) | 1.5x10⁹ | 5.5x10⁸ |
| Survival rate (%) | 66.4 | 72.4 |

### (3) Investigation of intestinal adherence

To evaluate the degree of adherence to intestinal cells of the *L. plantarum* LMT17-31 strain, a Caco-2 cell line (KCLB 30037.1) of the human epithelial colorectal adenocarcinoma cells purchased from the Korea Cell Line Bank was used. The Caco-2 cells were inoculated into a Dulbecco's modified Eagle's medium (DMEM; Gibco, USA) supplemented with 10 % fetal bovine serum (FBS) (Gibco, USA) under conditions of 5 % CO₂ and 37 °C to reach 7×10⁴ cells/100 µl. The cells were cultured to form a cellular monolayer in a 96-well plate (Corning, USA).

Separately, the LMT17-31 strain cultured in the MRS broth was washed with PBS, suspended in an antibiotic-free DMEM medium, and then added at a concentration of 1×10⁷ CFU to the Caco-2 cells constituting the cellular monolayer. The cells were cultured for 2 hours under conditions of 5 % CO₂ and 37 °C. To remove cells that failed to adhere to the Caco-2 cells, a washing process was performed thereon by using PBS 5 times, and 100 µl of 0.1 % Triton X-100 was used to detach the adhered cells that were then spread on an MRS solid medium. After culturing the cells at 37 °C for 24 hours, the number of colonies on the plate medium was counted to investigate the intestinal adherence of the LMT17-31 strain.

Table 5 shows the number of *L. plantarum* LMT17-31 strain adhered to the intestinal epithelial cells. As shown in Table 5, the novel *L. plantarum* LMT17-31 strain of the present invention showed about 2 % of adherence, and the comparative *L. plantarum* KCTC3108 strain showed about 1 % of adherence, to the Caco-2 intestinal epithelial cells.

**Table 5**

| | LMT17-31 | KCTC3108 |
|---|---|---|
| Number of strains treated (10⁷ CFU) | 1.2 | 0.8 |
| Number of bacteria adhered (10⁴ CFU) | 22.1 | 10.3 |
| Adhesion rate (%) | 1.87 | 1.32 |

### Example 3: Evaluation of anti-tumor efficacy of L. plantarum LMT17-31 strain

### 1. Induction of mouse model for tumor and administration of L. plantarum LMT17-31 strain

C57BL/6 mice (male, weight: 20 g to 22 g) used for induction of a mouse model for tumor were purchased from Orient Bio INC., and were acclimated to the environment for 1 week prior to the start of an experiment. 2.5×10⁵ MC38 cells derived from C57BL/6 murine colon adenocarcinoma cells were injected into the subcutaneous tissue of the mouse back, and after 1 week of the tumor injection, the mice were separated into groups based on the tumor size (50 mm³ to 70 mm³). For 2 weeks after the group separation, the *L. plantarum* LMT17-31 strain was orally administered with PBS containing 1×10⁹ CFU strain per mouse by utilizing a zonde once every other day. As a positive control group, an anti-PD1 antibody (clone number: RMP1-14, Manufacturer: Bioxcell, product name: InvivoMAb anti-mouse PD-1) was intraperitoneally administered twice a week at 10 mg per kg of mouse weight. As a negative control group, PBS was orally administered. Regarding compositions of the experimental groups, a total of 4 groups with 8 animals in each group were prepared as shown in Table 6.

**Table 6**

| | |
|---|---|
| Group 1 | PBS Control group |
| Group 2 | Group treated with anti-PD1 antibody |
| Group 3 | Group treated with *L. plantarum* LMT17-31 |
| Group 4 | Group treated with anti-PD1 antibody + LMT17-31 in combination |

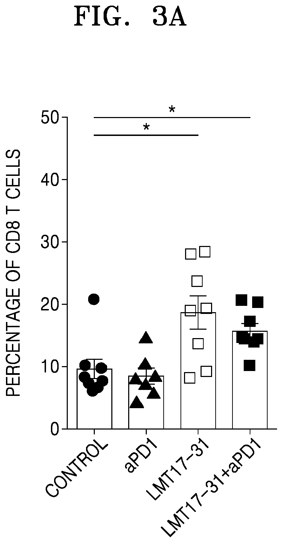

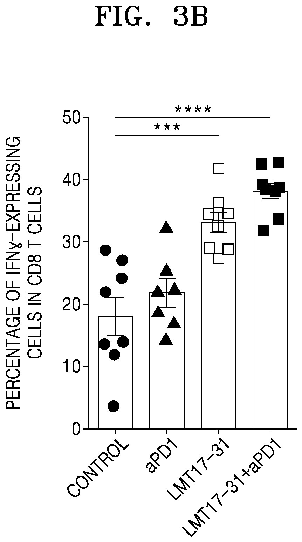

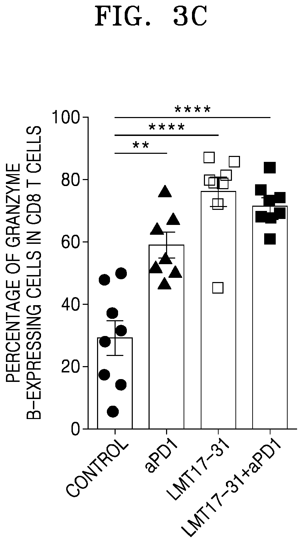

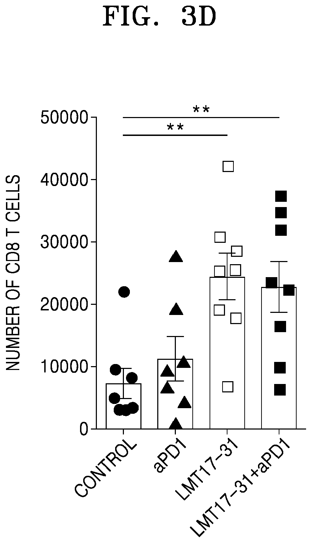

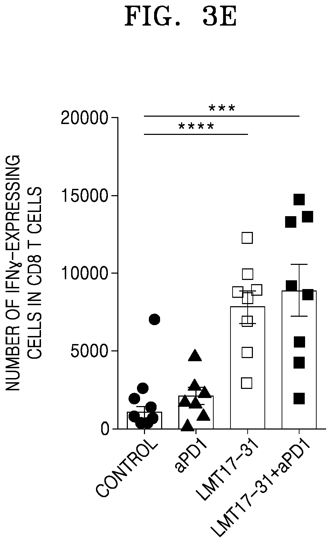

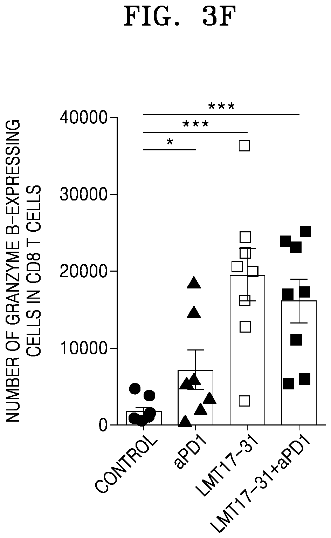

The tumor size was measured until day 23 after the MC38 tumor cell line was injected into the mice, and the mice were sacrificed by using carbon dioxide to extract the tumor. Then, analysis on tumor-infiltrating immune cells was performed. The analysis results are shown in FIGS. 3A, 3B, 3C, 3D, 3E, and 3F. FIGS. 3A, 3B, 3C, 3D, 3E, and 3F are diagrams showing the results of analyzing CD8 T cells infiltrating into the tumor and secretion of cytokines therefrom after the *L. plantarum* LMT17-31 strain was administered to the mice having the tumor. The MC38 tumor cell line includes mouse colon adenocarcinoma cells induced by subcutaneous injection of dimethylhydrazine into C57BL/6 mice. The induced mouse model for tumor used in Examples herein was prepared by transplanting the MC38 tumor cells into the subcutaneous tissue of the mouse, indicating that the effect of orally administered bacteria can exhibit on inhibition of the cancer growth in the subcutaneous tissue and that the bacteria can affect not only a cancer related to the intestines but various solid carcinomas.

### 2. Evaluation of antitumor efficacy according to administration of L. plantarum LMT17-31 strain

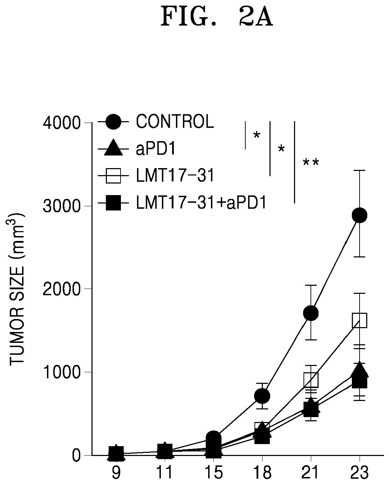

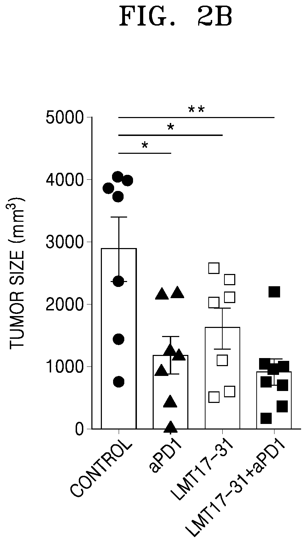

FIGS. 2A and 2B are diagrams showing the results of measuring the tumor size after the *L. plantarum* LMT17-31 strain was administered to the mice having the tumor. The administration was performed according to Section 1. In detail, the rumor size was measured twice a week from day 7 to day 23 after the injection of the tumor cell line. To accurately measure the tumor size, the long axis and the short axis of the tumor were measured by using a vernier caliper, and the tumor size was calculated by the formula of 'long axis x (short axis)²/2'. In FIGS. 2A and 2B, PBS indicates a group administered with PBS as a negative control group, aPD1 indicates a group administered with anti-PD1 antibody as a positive control group, LMT17 indicates a group administered with the LMT17 strain as an experimental group, and LMT17-31+aPD1 indicates a group administered with the LMT17-31 strain and anti-aPD1 antibody in combination as an experimental group. FIG. 2A shows the tumor size according to the number of days after the cell injection, and FIG. 2B shows the tumor size on day 23 after the cell injection. In FIG. 2A, the numbers on the horizontal axis represent days on which the tumor size was measure, and that is, days elapsed after the administration of the tumor cells. In FIG. 2B, the bars indicate all tumor sizes for each subject on day 23 after the cell injection. As shown in FIGS. 2A and 2B, statistically significant tumor growth inhibition was observed in both the group administered with the LMT17-31 strain alone and the group administered with the LMT17-31 strain and anti-PD1 antibody in combination.

### 3. Analysis of tumor-infiltrating immune cell and evaluation of functionality of immune cell

CD8 T cells that are tumor-infiltrating immune cells are important indicators of anticancer responses, and interferon gamma and granzyme B that are secreted by the CD8 T cells are functional cytokines showing the activation ability of immune cells. FIG. 3 shows the results of analyzing the CD8 T cells infiltrating into the tumor and the secretion of cytokines secreted by the CD8 T cells, after the *L. plantarum* LMT17-31 strain was administered to the mice having the tumor. The administration was performed according to Section 1. The tumor was excised on day 23 after the administration. The excised tumor was separated into single cells by using an RPMI1640 medium supplemented with 50 ug/ml of Liberase and 40 ug/ml of DNase I and utilizing a cell strainer. To observe the patterns of producing interferon gamma by T cells, the separated single cells were stimulated for 4 hours with 50 ng/ml of phorbol 12-myristate 13-acetate (PMA) and 500 ng/ml of ionomycin in an RPMI1640 medium supplemented with 10 % FBS. The PMA and ionomycin substances are substances that cause signal stimulation to activate T cells, and play the same role as the activation mechanism of T cells upon actual antigens, and thus can provide an environment in which an immune response seems to occur. Ionomycin as a Ca2+ ionophore increases a level of protein kinase C (PKC). In the case of PMA, by phosphorylating PKC, PMA synergizes for targeting CD4 T cells and CD8 T cells. After the stimulation, the cells were stained with the antibodies shown in Table 7 for the analysis on the immune cells and the identification of the production of interferon gamma, and then analyzed by using a CANTO II flow cytometry apparatus.

**Table 7**

| Description | Target | Color |
|---|---|---|
| Surface marker | CD45 | PE-Cy7 |
| | TCRβ | APC-Cy7 |
| | CD4 | PerCP-Cy5.5 |
| | CD8 | Pacific blue |
| Intracellular staining | IFNγ | FITC |
| | Granzyme B | APC |

FIGS. 3A, 3B, and 3C show the percentage of CD8 T cells in the tumor-infiltrating T cells, the percentage of IFNγ-expressing cells in the CD8 T cells, and the percentage of granzyme B-expressing cells in the CD8 T cells, respectively. FIGS. 3D, 3D, and 3F show the total number of tumor-infiltrating CD8 T cells, the number of IFNγ-expressing cells in the CD8 T cells, and the number of granzyme B-expressing cells in the CD8 T cells, respectively. As shown in FIG. 3, the percentage and number of the tumor-infiltrating CD8 T cells increased significantly in the group administered with the LMT17-31 strain, and the group administered with the anti-PD1 antibody and LMT17-31 strain in combination, compared to the PBS-treated group. Also, regarding the percentage and number of the CD8 T cells that produce activating factors, i.e., interferon gamma and granzyme B, a significant increase was shown in the group treated with the LMT17-31 strain, and the group treated with the anti-PD1 antibody and LMT17-31 strain in combination, compared to the PBS control group.

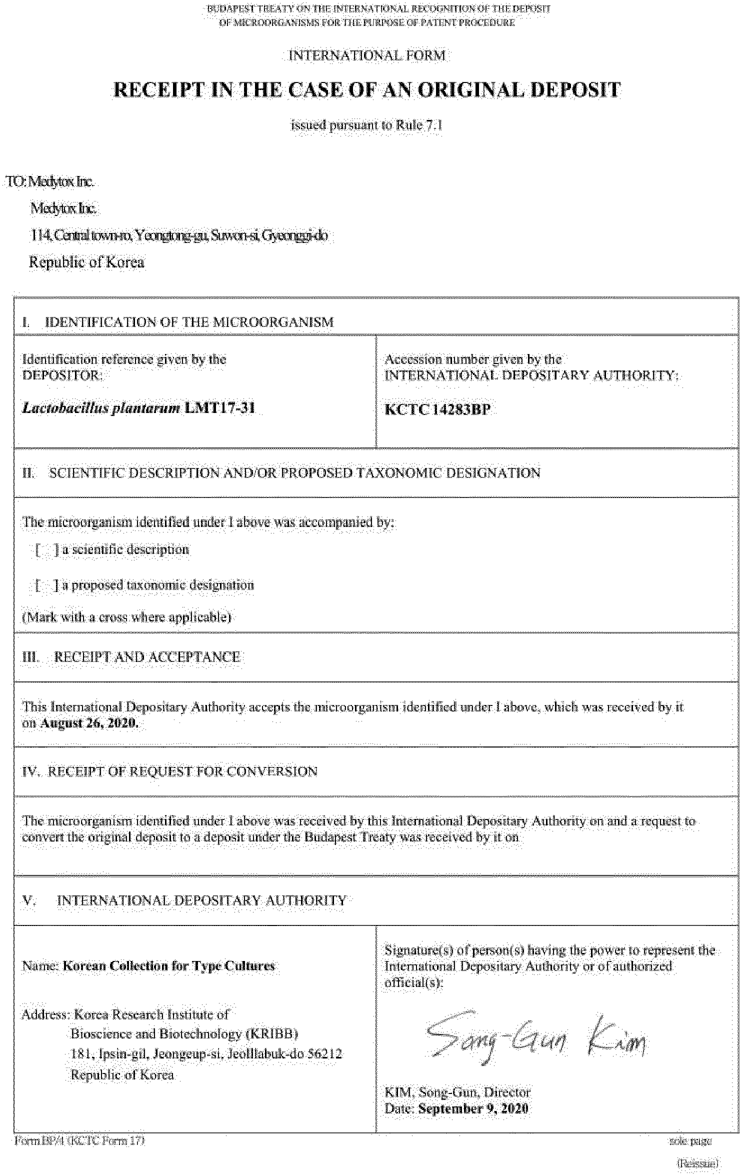
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Medytox Inc.
Medytox Inc.
114, Central town-ro, Yeongtong-gu, Suwon-si, Gyeonggi-do
Republic of Korea

I. IDENTIFICATION OF THE MICROORGANISM
Identification reference given by the
DEPOSITOR:

Lactobacillus plantarum LMT17-31 | Accession number given by the
INTERNATIONAL DEPOSITARY AUTHORITY:

KCTC 14283BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 26, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: September 9, 2020
Form BP/4 (KCTC Form 17)                                                                                    sole page
(Reissue)

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Medytox Inc.
Medytox Inc.
114, Central town-ro, Yeongtong-gu, Suwon-si, Gyeonggi-do
Republic of Korea

I. IDENTIFICATION OF THE MICROORGANISM
Identification reference given by the DEPOSITOR: | Accession number given by the INTERNATIONAL DEPOSITARY AUTHORITY:
Lactobacillus plantarum LMT17-31 | KCTC 14283BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 26, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures | Signature(s) of person(s) having the power to represent the International Depositary Authority or of authorized official(s):
Address: Korea Research Institute of
Bioscience and Biotechnology (KRIBB)
181, Ipsin-gil, Jeongeup-si, Jeollabuk-do 56212
Republic of Korea | Song-Gun Kim
KIM, Song-Gun, Director
Date: September 9, 2020

Form BP/4 (KCTC Form 17) sole page
(Reissue)

## Claims

1. A microorganism as *Lactobacillus plantarum* LMT17-31 (Accession No.: KCTC-14283BP) having anti-tumor activity.

2. A pharmaceutical composition for preventing or treating a cancer comprising, as an active ingredient, the microorganism of claim 1 or a culture or extract thereof.

3. The composition of claim 2, wherein the cancer is a solid cancer.

4. The composition of claim 2, wherein the composition is administered in combination with an immune checkpoint inhibitor.

5. The composition of claim 4, wherein the immune checkpoint inhibitor is a CTLA4 inhibitor, a PD-1 inhibitor, or a PD-L1 inhibitor.

6. A food composition for preventing or ameliorating a cancer comprising, as an active ingredient, the microorganism of claim 1 or a culture or extract thereof.
